# EUROPEAN PATENT APPLICATION

(11) **EP 3 872 233 A1**
(43) Date of publication of application: **01.09.2021**
(21) Application number: 20159566.7
(22) Date of filing: 26.02.2020
(51) Int. Cl.: C25B 1/04, C07C 1/12, F01K 23/00

(54) **METHOD FOR STORING ENERGY AND ENERGY STORING SYSTEM**

(71) Applicant: Doosan Lentjes GmbH, 40880 Ratingen Nordrhein-Westfalen (DE)
(72) Inventor: KEHRMANN, Kai Matthias, 44577 Castrop-Rauxel (DE); EINICKE, Clemens, 40699 Erkrath (DE)
(74) Representative: Feucker, Max Martin

(57) **Abstract**

The present invention relates to a method for temporary storing electrical energy, comprising at least the following steps:
- Operating an electrolysis unit (2) to produce hydrogen and oxygen,
- Using primary electrical energy to operate the electrolysis unit (2),
- Operating a hydrocarbons reactor unit (3) to produce hydrocarbons and water,
- Operating a secondary energy generator (4) to produce secondary energy and carbon oxide,
- Providing the hydrogen produced by the electrolysis unit (2) to the hydrocarbons reactor unit (3),
a1) Compressing the oxygen produced by the electrolysis unit (2) and storing the compressed oxygen,
a2) Expanding the compressed oxygen and supplying the expanded oxygen to the secondary energy generator (4),
b1) Compressing the hydrocarbons produced by the hydrocarbons reactor unit (3) and storing the compressed hydrocarbons,
b2) Expanding the compressed hydrocarbons and supplying the expanded hydrocarbons to the secondary energy generator (4),
c1) Compressing the carbon oxide produced by the secondary energy generator (4) and storing the compressed carbon oxide.

## Description

The present invention relates to a method for storing energy in a CO₂ neutral manner and to a respective energy storing system, wherein primary electrical energy from a primary electrical energy source (such as renewable energy sources) is converted to gases (such as methane and oxygen) and wherein the gases are combusted to produce secondary energy, such as secondary electrical energy and/or heat, on demand. The system comprises an electrolysis unit for producing (pure) hydrogen (H₂) and (pure) oxygen (O₂), a hydrocarbons reactor unit for producing hydrocarbons (i.e. methane CH₄ and/or methanol CH₃O) and water and a secondary energy generator for producing secondary electrical energy, heat and carbon oxide (such as carbon monoxide CO and/or carbon dioxide CO₂, in particular comprised in flue gas, which additionally may comprise CH₄, O₂ and/or H₂) and water (H₂O). The electrolysis unit is operated by supplying the primary electrical energy and is connected to the hydrocarbons reactor unit to supply the hydrogen produced by the electrolysis unit to the hydrocarbons reactor unit. The electrolysis unit is also connected to the secondary energy generator to supply the oxygen produced by the electrolysis unit to the secondary energy generator. The hydrocarbons reactor is connected to the secondary energy generator to supply the hydrocarbons produced by the hydrocarbons reactor unit to the secondary energy generator. The secondary energy generator is connected to the hydrocarbons reactor unit to supply the carbon oxide produced by the secondary energy generator to the hydrocarbons reactor unit.

In case there is a surplus of electrical energy for example from renewable energy sources, the system can be used to produce and store oxygen and hydrocarbons in respective storage vessels. In case the demand for electrical energy is higher than the electrical energy that can by supplied by the renewable energy sources or other sources, the stored hydrocarbons and oxygen can be used to operate the secondary energy generator, while the carbon oxide produced by the secondary energy generator can be stored in order to be used to operate the hydrocarbons reactor.

WO 2013/029701 A1 discloses an autonomous power supply system for the field of building services, which is to be connected to a primary energy source, a natural gas network or an external H₂/CO₂ source.

A further system is known from DE 10 2012 214 907 B4, in which the secondary energy generator comprises a heat recovery steam generator to produce the secondary electrical energy. Such heat recovery steam generator is not efficient, when electrical energy is demanded suddenly.

EP 2 426 236 B1 discloses a respective system and a method to operate the system, in which the secondary energy generator comprises a gas turbine or a steam turbine. Gas turbines and steam turbines are embodied to run at a steady load for a long time. As it takes a comparatively long time to run up gas turbines and steam turbines to a steady load, such gas turbines and steam turbines are not able to react on a sudden demand for electrical energy. Additionally, gas turbines and steam turbines run efficiently only in big scale systems, so that they can not be used cost efficiently in comparatively small local systems near the location where renewable energy is produced. Furthermore, it is disclosed that the hydrocarbons and the oxygen are provided to the combustion chamber in a compressed/pressurised state. Accordingly, it is an object of the present invention to avoid the drawbacks of the prior art and to provide an energy storing system and a method for temporary storing electrical energy, which are more efficient.

The object is achieved by a method and an energy storage system with the features of the respective independent claim. Preferred embodiments of the method and the system are described in the dependent claims and in the description, wherein single features of the preferred embodiments can be combined with each other in a technical meaningful manner. In particular, features described with respect to the system apply to the method and vice versa.

The object is in achieved in that
- the oxygen produced by the electrolysis unit is compressed and the compressed oxygen is stored,
- the compressed oxygen is expanded and subsequently supplied to the secondary energy generator,
- the hydrocarbons produced by the hydrocarbons reactor unit is compressed and the compressed hydrocarbons is stored,
- the compressed hydrocarbons is expanded and subsequently supplied to the secondary energy generator, and
- the carbon oxide produced by the secondary energy generator is compressed and the compressed carbon oxide is stored.

Accordingly, the energy storing system comprises
- an oxygen compressor connected to the electrolysis unit for compressing the oxygen produced by the electrolysis unit,
- an oxygen storage vessel connected to the oxygen compressor for storing the compressed oxygen,
- an oxygen expander connected to the oxygen storage vessel and to the secondary energy generator for expanding the compressed oxygen and supplying the expanded oxygen to the secondary energy generator,
- a hydrocarbons compressor connected to the hydrocarbons reactor unit for compressing the produced hydrocarbons,
- a hydrocarbons storage vessel connected to the hydrocarbons compressor for storing the compressed hydrocarbons,
- a hydrocarbons expander connected to the hydrocarbons storage vessel and to the secondary energy generator for expanding the compressed hydrocarbons and supplying the expanded hydrocarbons to the secondary energy generator,
- a carbon oxide compressor connected to the secondary energy generator for compressing the carbon oxide produced by the secondary energy generator, and
- a carbon oxide storing vessel connected to the carbon oxide compressor for storing the compressed carbon oxide, the carbon oxide storing vessel also being connected to the hydrocarbons reactor unit.

As the CO₂ produced in the secondary energy generator is kept in the quasi-closed circuit to operate the hydrocarbons reactor unit, the system is CO₂ neutral.

Additionally, as pure oxygen is used to operate the secondary energy generator the exhaust gases do not need to be cleaned avoiding the effort for a flue (exhaust) gas cleaning.

Accordingly, the invention suggests that the hydrocarbons and the oxygen produced during a surplus of primary electrical energy are stored in a compressed state, while the hydrocarbons and the oxygen each are expanded before supplied to a combustion chamber of the secondary energy generator, when there is a need for secondary energy, such as secondary electrical energy and/or heat. At the same time, when the secondary energy is produced the carbon oxide (i.e. CO and/or CO₂) produced is compressed and stored in a compressed state. This way, the expander for the hydrocarbons and/or the expander for the oxygen might be (thermally) coupled with the compressor for the carbon oxide to use the heat which will be produced during compression of the carbon oxide to heat the respective expander . Alternatively or additionally, the expander for the hydrocarbons and/or the expander for the oxygen might be thermally coupled with the secondary energy generator, to use the heat produced during combustion to heat the respective expander. On the other hand, this way the flue gases of the secondary energy generator can be cooled to separate the carbon oxide and water. Thereby, the overall efficiency of storing electrical energy is enhanced.

Furthermore, the expanded hydrocarbons and the expanded oxygen can be provided (as a mixture or separately) to a combustion chamber of the secondary energy generator in a more or less unpressurized state. Accordingly, the secondary energy generator comprises a combustion chamber, to which the expanded hydrocarbons and the expanded oxygen is provided.

When the compressed oxygen and/or the compressed hydrocarbons are expanded the hydrocarbons and the oxygen and also the hydrocarbons expander as well as the oxygen expander are cooled due to the expansion, which seems to be the reason, why such expansion was not taught in the prior art. With the present invention, such cooling of the expanded oxygen and/or expanded hydrocarbons or the respective expanders can be reduced or avoided, for which energy within the system can be used.

With the present invention, on the one hand the need of external energy sources for heating of the expanded gases as for example by burning of natural gas or by heat storage will be avoided and on the other hand, the stored potential energy from the compressed gases will be recovered by the expander.

For example, the thermal energy caused when compressing the carbon oxide may be provided to the expander for expanding the hydrocarbons and/or to the expander for expanding the oxygen. In this case, the carbon oxide compressor is thermally connected with the oxygen expander and/or with the hydrocarbons expander, so that thermal energy caused in the carbon oxide expander is used to heat the oxygen expander and/or the hydrocarbons expander.

For such thermal energy transfer the carbon oxide compressor and/or a line connecting the carbon oxide compressor to the carbon oxide storing vessel may comprise a carbon oxide compressor heat exchanger.

In order to transfer thermal energy (heat) to the oxygen expander the oxygen expander and/or a line connecting the oxygen expander to the secondary energy generator comprises an oxygen expander heat exchanger.

In order to transfer thermal energy to the hydrocarbons expander, the hydrocarbons expander and/or a line connecting the hydrocarbons expander to the secondary energy generator comprises a hydrocarbons expander heat exchanger.

In order to thermally couple the carbon oxide compressor to the oxygen expander and/or hydrocarbons expander the carbon oxide compressor heat exchanger may be connected to the oxygen expander heat exchanger and/or hydrocarbons expander heat exchanger.

In a further embodiment thermal energy caused by operating the secondary energy generator is provided to the expansion of the compressed oxygen and/or to the expansion of the compressed hydrocarbons. In this case the secondary energy generator and/or an exhaust line connecting the secondary energy generator to the carbon oxide compressor comprises a secondary energy generator heat exchanger, which is in connection with the oxygen expander heat exchanger and/or hydrocarbons expander heat exchanger. This way, the secondary energy generator is connected with the oxygen expander and/or with the hydrocarbons expander, so that thermal energy caused by operating the secondary energy generator is used to heat the oxygen expander and/or the hydrocarbons expander.

It is also possible, that by expanding the compressed oxygen and/or by expanding the compressed hydrocarbons electrical energy is generated, for which the oxygen expander and/or the hydrocarbons expander is embodied to produce electrical energy. Accordingly, the oxygen expander and/or the hydrocarbons expander is/are connected to a generator in a known manner. A generator may be provided for each expander or a common generator may be provided for the oxygen expander and the hydrocarbons expander.

It might also be possible that the oxygen expander and/or the hydrocarbons expander is/are mechanically coupled to the carbon oxide compressor so that mechanical energy produced with the expansion of the hydrocarbons and/or with the expansion of the oxygen is directly transferred to the carbon oxide compressor for compressing the carbon oxide.

As described above there are multiple ways how the energy generated by the expansion of the compressed oxygen and/or by the expansion of the compressed hydrocarbons can be used further. This way, it is possible that the hydrocarbons and the oxygen can be stored in a compressed state, while the energy for compressing the oxygen and the hydrocarbons is at least partly recovered and directly used in the further process.

Accordingly, preferably the oxygen expander and/or the hydrocarbons expander is thermally, mechanically or electrically connected to the carbon oxide compressor.

Principally, the compressed carbon oxide may be supplied in a pressurized (compressed) state to the hydrocarbons reactor unit. Alternatively, in a further embodiment of the invention the compressed carbon oxide stored in the carbon oxide storing vessel may be expanded (at least to a lower level) and subsequently supplied to the hydrocarbons reactor unit. In this case the system comprises a carbon oxide expander connected to the carbon oxide storing vessel and to the hydrocarbons reactor unit for expanding the compressed carbon oxide and supplying the expanded hydrocarbons to the hydrocarbons reactor unit.

In case a carbon oxide expander is embodied, the carbon oxide expander may be thermally, mechanically or electrically connected to the hydrocarbons compressor and/or to the oxygen compressor, so that energy can be kept in the system. In this case the carbon oxide expander and/or a line connecting the carbon oxide expander to the hydrocarbons reactor unit may comprise a carbon oxide expander heat exchanger, which is connected to an oxygen compressor heat exchanger and/or hydrocarbons compressor heat exchanger.

The storage vessel for the oxygen, hydrocarbons and carbon oxide may be any suitable storage vessel, such as tanks, already existing pipe lines connected to the tanks or caverns. Alternative or in addition, the carbon oxide and the hydrocarbons can be stored in common or connected vessel.

In a preferred embodiment the secondary energy generator comprises a piston engine, wherein the expanded oxygen and the expanded hydrocarbons produced by the hydrocarbons reactor are combusted to operate the piston engine. For example, the oxygen and the hydrocarbons can be injected independently or directly as a mixture into the combustion chamber (cylinder) of the piston engine, so that the piston of the engine is driven by the expansion of the combusted gases. Alternatively, the oxygen and hydrocarbons can be combusted together and the thermal energy of the combustion process is used to operate a stirling engine. The piston engine comprises or is connected to an electrical generator connected to the piston engine. A Wankel engine with a rotating piston is also considered a piston engine. A piston engine can run up much faster than the turbines of a heavy industry system. Additionally, comparatively small piston engines can work efficiently, so that the system can be set up locally near the source of the renewable energy or the energy consumer.

The secondary energy generator (piston engine) may comprise a recirculation line to recirculate part of the combustion gases, in particular (pure) carbon oxide produced by operating the secondary energy generator (piston engine), back to the combustion process. The recirculation line may also be connected to the carbon oxide expander, so that in particular when starting the combustion process expanded carbon oxide is provided to the combustion process (together with the hydrocarbons and the oxygen) for controlling the combustion parameters. This way the combustion process of the (initially pure) hydrocarbons and the (initially pure) oxygen can be optimized. As no air is used for the combustion process, nitrogen oxides as components of the combustion gases are avoided.

Furthermore, the system may be operated/embodied as closed loop system to which only the primary energy is provided during operation. This means, that all the fluids produced by the elements of the system (electrolysis unit, hydrocarbons reactor, secondary energy generator) are kept in the system and are not provided to external processes and/or are received from external processes. Of course, this does not rule out that the fluids are provided after installation of the system and/or that the fluids are filled up in case of leakage or inefficiencies.

In a further embodiment of the invention the water produced by the secondary energy generator and/or the water produced by the hydrocarbons reactor may be supplied to the electrolysis unit. Accordingly, the secondary energy generator and/or the hydrocarbons reactor are connected to the electrolysis unit. Also in this regard, a vessel for temporary storing the water may be provided.

The electrolysis unit is preferably operated at a temperature between 50°C and 1000°C. In particular, in case of an alkaline electrolysis the temperature is between 60°C and 100°C and in case of a high temperature electrolysis the temperature is between 100°C and 1000°C.

The electrolysis unit is preferably operated with a pressure between 10 bar and 200 bar, preferably between 20 bar and 90 bar.

The hydrocarbons reactor unit is preferably operated at a temperature between 1°C and 700°C, preferably between 200°C and 500°C.

The hydrocarbons reactor unit is preferably operated with a pressure between 1 bar and 200 bar, preferably between 10 bar and 90 bar.

The gas storage is operated between 1 bar and 300 bar, preferably between 50 bar and 210 bar.

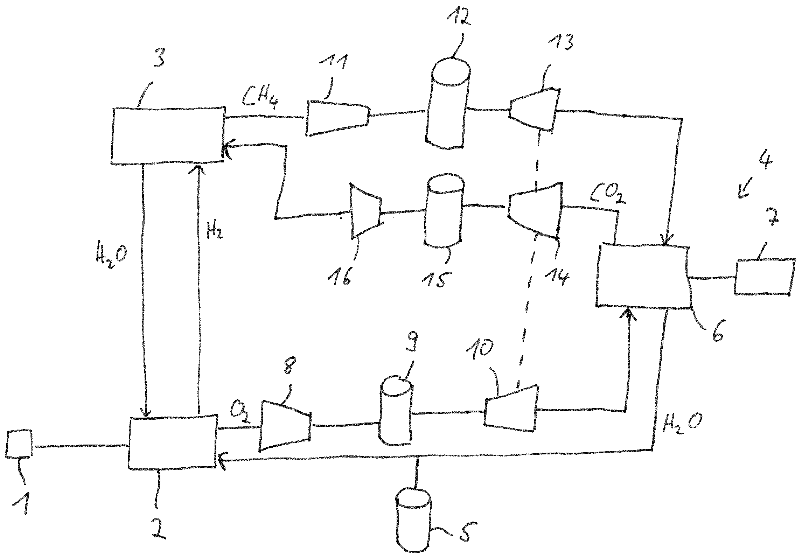

The invention and the technical background will now be explained with regard to the figure, which shows an exemplary embodiment of the invention.

The figure shows schematically a system which comprises an electrolysis unit 2, a hydrocarbons reactor unit 3 and a secondary energy generator 4, which is embodied by a piston engine 6 and an electrical generator 7 connected to the piston engine 6.

The electrolysis unit 2 is connected to a primary electrical energy source 1.

In operation, when there is a surplus of primary energy the electrolysis unit 2 produces pure hydrogen H₂ and pure oxygen O₂ from water H₂O.

The hydrogen H₂ produced by the electrolysis unit 2 is supplied to the hydrocarbons reactor unit 3, while the oxygen O₂ produced by the electrolysis unit 2 is compressed in oxygen compressor 8 and stored in oxygen storage vessel 9.

The hydrocarbons reactor unit 3 produces hydrocarbons (i.e. methane CH₄ and/or methanol CH₃O) by a catalytic reaction of hydrogen H₂ supplied from the electrolysis unit 2 and carbon dioxide CO₂ produced by the piston engine 6 and stored in a carbon oxide storage vessel 15. Before being supplied to the hydrocarbons reactor unit 3 the carbon dioxide is expanded in carbon oxide expander 16.

The hydrocarbons produced by the hydrocarbons reactor unit 3 is compressed in hydrocarbons compressor 11 and stored in hydrocarbons storage vessel 12.

When there is a shortage of primary energy, the system is run to operate the secondary energy generator 4 to produce secondary electrical energy.

The compressed hydrocarbons stored in the hydrocarbons storage vessel 12 is expanded in hydrocarbons expander 13 and the expanded hydrocarbons is supplied to the combustion chamber of the piston engine 6.

Also, the compressed oxygen stored in the oxygen storage vessel 9 is expanded in oxygen expander 10 and the expanded oxygen is supplied to the combustion chamber of piston engine 6 of the secondary energy generator 4.

The piston engine 6 is operated by combusting the oxygen O₂, the hydrocarbons and optionally the carbon oxide, which may be provided by a recirculating line, which may be connected to the carbon oxide expander 16. The hydrocarbons and the oxygen O₂ may be directly combusted in a cylinder of the piston engine in order to drive the piston directly by the expansion of the combusted gases. Alternatively, the piston engine may be embodied as stirling engine in which the thermal energy is used to drive the pistons. The torque produced by the piston engine 6 is transferred into electrical energy by the electrical generator 7.

The combustion gases, in particular carbon oxide (such as CO and/or CO₂), produced by the piston engine 6 is compressed by carbon oxide compressor 14 and stored in carbon oxide storage vessel 15.

When the secondary energy generator 4 is operated, heat produced by compressing the carbon oxide within the carbon oxide compressor 14 is supplied to the hydrocarbons expander 13 and to the oxygen expander 10, so that the hydrocarbons expander 13 and the oxygen expander 10 are heated by the thermal energy produced by compressing the carbon oxide.

In a not shown embodiment the oxygen expander 10 and the hydrocarbons expander 13 may be coupled to a generator for producing electrical energy. In a further embodiment the oxygen expander 10, the hydrocarbons expander 13 and the carbon oxide expander 16 may be mechanically coupled to the carbon oxide compressor 14. In an even further embodiment the oxygen expander 10 and the hydrocarbons expander 13 may be thermally coupled to the secondary energy generator 4, so that heat produced by the secondary energy generator 4 may be supplied to the oxygen expander 10 and the hydrocarbons expander 13. Furthermore, in a further embodiment the carbon oxide expander 16, the oxygen expander 10 and the hydrocarbon expander 13 may be thermally coupled with the secondary energy generator 4 so that the flue gas from the secondary energy generator 4 can be cooled to separate the flue gas into water and carbon oxide.

The water H₂O produced by secondary energy generator 4 and/or by the hydrocarbons reactor 3 may be stored in a water storage vessel 5 for reuse.

### Reference numbers

- 1: primary electrical energy source
- 2: electrolysis unit
- 3: hydrocarbons reactor unit
- 4: secondary energy generator
- 5: water storage vessel
- 6: piston engine
- 7: electrical generator
- 8: oxygen compressor
- 9: oxygen storage vessel
- 10: oxygen expander
- 11: hydrocarbons compressor
- 12: hydrocarbons storage vessel
- 13: hydrocarbons expander
- 14: carbon oxide compressor
- 15: carbon oxide storage vessel
- 16: carbon oxide expander

## Claims

1. Method for temporary storing energy, comprising at least the following steps:
- Operating an electrolysis unit (2) to produce hydrogen and oxygen,
- Using primary electrical energy to operate the electrolysis unit (2),
- Operating a hydrocarbons reactor unit (3) to produce hydrocarbons and water,
- Operating a secondary energy generator (4) to produce secondary energy and carbon oxide,
- Providing the hydrogen produced by the electrolysis unit (2) to the hydrocarbons reactor unit (3),
a1) Compressing the oxygen produced by the electrolysis unit (2) and storing the compressed oxygen,
a2) Expanding the compressed oxygen and supplying the expanded oxygen to the secondary energy generator (4),
b1) Compressing the hydrocarbons produced by the hydrocarbons reactor unit (3) and storing the compressed hydrocarbons,
b2) Expanding the compressed hydrocarbons and supplying the expanded hydrocarbons to the secondary energy generator (4),
c1) Compressing the carbon oxide produced by the secondary energy generator (4) and storing the compressed carbon oxide,
- Using the provided hydrogen and the carbon oxide to operate the hydrocarbons reactor unit (3) and
- Using the supplied hydrocarbons and the supplied oxygen to operate the secondary energy generator (4).

2. Method according to claim 1, wherein thermal energy caused in step
c1) is provided to step a2) and/or b2) and/or
thermal energy caused by operating the secondary energy generator (4) is provided to step a2) and/or b2).

3. Method according to claim 1 or 2, wherein electrical energy is generated in step a2) and/or b2).

4. Method according to one of the preceding claims, comprising the steps of
c2) Expanding the compressed carbon oxide,
c3) Supplying the expanded carbon oxide to the hydrocarbons reactor unit (3).

5. Method according to one of the preceding claims, wherein the expanded hydrocarbons and the expanded oxygen are provided to a combustion chamber of the secondary energy generator (4).

6. Method according to one of the preceding claims, wherein the system is operated as closed loop to which only the primary energy is provided.

7. Energy storing system, comprising:
- an electrolysis unit (2) for producing hydrogen and oxygen,
- a hydrocarbons reactor unit (3) for producing hydrocarbons and water, wherein the electrolysis unit (2) is connected to the hydrocarbons reactor unit (3) to supply the hydrogen produced by the electrolysis unit (2) to the hydrocarbons reactor unit (3) and
- a secondary energy generator (4) for producing secondary energy and carbon oxide,
a1) an oxygen compressor (8) connected to the electrolysis unit (2) for compressing the oxygen produced by the electrolysis unit (2),
a2) an oxygen storage vessel (9) connected to the oxygen compressor (8) for storing the compressed oxygen,
a3) an oxygen expander (10) connected to the oxygen storage vessel (9) and to the secondary energy generator (4) for expanding the compressed oxygen and supplying the expanded oxygen to the secondary energy generator (4),
b1) a hydrocarbons compressor (11) connected to the hydrocarbons reactor unit (3) for compressing the produced hydrocarbons,
b2) a hydrocarbons storage vessel (12) connected to the hydrocarbons compressor (11) for storing the compressed hydrocarbons,
b3) a hydrocarbons expander (13) connected to the hydrocarbons storage vessel (12) and to the secondary energy generator (4) for expanding the compressed hydrocarbons and supplying the expanded hydrocarbons to the secondary energy generator (4),
c1) a carbon oxide compressor (14) connected to the secondary energy generator (4) for compressing the carbon oxide produced by the secondary energy generator (4),
c2) a carbon oxide storing vessel (15) connected to the carbon oxide compressor (14) for storing the compressed carbon oxide, the carbon oxide storing vessel (15) being connected to the hydrocarbons reactor unit (3),

8. System according to claim 7, wherein the carbon oxide compressor (14) is connected with the oxygen expander (10) and/or with the hydrocarbons expander (13), so that thermal energy caused in the carbon oxide compressor (14) is used to heat the oxygen expander (10) and/or the hydrocarbons expander (13).

9. System according to claim 7 or 8, wherein the secondary energy generator (4) is connected with the oxygen expander (10) and/or with the hydrocarbons expander (13), so that thermal energy caused by operating the secondary energy generator (4) is used to heat the oxygen expander (10) and/or the hydrocarbons expander (13).

10. System according to one of claims 7 to 9, wherein the oxygen expander (10) and/or the hydrocarbons expander (13) is embodied to produce electrical energy.

11. System according to one of claims 7 to 10, comprising c3) a carbon oxide expander (16) connected to the carbon oxide storing vessel (15) and to the hydrocarbons reactor unit (3) for expanding the compressed carbon oxide and supplying the expanded hydrocarbons to the hydrocarbons reactor unit (3).

12. System according to one of claims 7 to 11, wherein the oxygen expander (10) and/or a line connecting the oxygen expander (10) to the secondary energy generator (4) comprises an oxygen heat exchanger.

13. System according to one of claims 7 to 12, wherein the hydrocarbons expander (13) and/or a line connecting the hydrocarbons expander (13) to the secondary energy generator (4) comprises a hydrocarbons heat exchanger.

14. System according to one of claims 9 to 13, wherein the secondary energy generator (4) and/or an exhaust line connecting the secondary energy generator (4) to the carbon oxide compressor (14) comprises a secondary energy generator heat exchanger.

15. System according to one of claims 9 to 13, wherein the carbon oxide compressor (14) and/or a line connecting the carbon oxide compressor (14) to the carbon oxide storing vessel (15) comprises a carbon oxide heat exchanger.

16. System according to one of claims 9 to 13, wherein the secondary energy generator (4) comprises a piston engine (6), wherein the oxygen and the hydrocarbons are combusted to operate the piston engine (6).
